# EUROPÄISCHE PATENTSCHRIFT

(11) **EP 0 453 656 B1**
(45) Veröffentlichungstag und Bekanntmachung des Hinweises auf die Patenterteilung: **17.01.1996**
(21) Anmeldenummer: 90125381.5
(22) Anmeldetag: 22.12.1990
(51) Int. Cl.: C12N 9/24

(54) **Verfahren zur Herstellung lignolytischer Enzyme mittels Phanerochaete Chrysosporium**
Process for production of lignolytic enzymes by phanerochaete chrysosporium
Procédé de production d'enzymes lignolytiques par utilisation de phanerochaete chrysosporium

(30) Priorität: 21.04.1990 DE 4012743
(43) Veröffentlichungstag der Anmeldung: 30.10.1991
(73) Patentinhaber: LIGNOZYM GESELLSCHAFT ZUR HERSTELLUNG UND ZUM VERTRIEB VON ENZYMEN mbH, D-52499 Baesweiler (DE)
(72) Erfinder: Call, Hans-Peter, Dr., D-52531 Übach-Palenberg (DE)
(74) Vertreter: Fitzner, Ulrich, Dr.

(56) Entgegenhaltungen:
- EP-A- 0 300 867
- JOURNAL OF BIOTECHNOLOGY, Band 8, 1988, Amsterdam H. JANSHEKAR et al. "Cultivation of Phanerochaete chrysosporium and production of lignin peroxidases in submerged stirred tank reactors" Seiten 97-112

## Beschreibung

Die vorliegende Erfindung betrifft ein Verfahren zur Herstellung lignolytischer Enzyme mittels Phanerochaete chrysosporium.

In den letzten 10 Jahren ist vielfach versucht worden, den Weißfäulepilz Phanerochaete chrysosporium im industriellen Maßstab zur Erzeugung lignolytischer Enzyme einzusetzen. So gibt es Versuche im Rührfermenter (H. Janshekar und A. Fiechter, Journal of Biotechnology, 8 (1988), 97-112). Diese Versuchen waren jedoch wenig erfolgreich, weil im Rührfermenter die von Phanerochaete chrysosporium gebildeten Pellets immer wieder zerschlagen werden. Da aber Phanerochaete chrysosporium nur dann Enzyme produziert, wenn es in Pelletform oder als Immobilisat vorliegt, läßt sich im Rührfermenter eine optimale Enzymproduktion nicht erreichen.

Darüber hinaus sind in der Bioverfahrenstechnik Reaktoren speziell für empfindliche Zellen entwickelt worden. So ist ein sogenannter Vibromischer entwickelt worden, welcher sich sogar für empfindliche Tier- und Pflanzenzellen eignet (Einsele, Finn, Samhaber: Mikrobiologische und biochemische Verfahrenstechnik, Weinheim 1985, S. 150; Einsele: Chem.Ing.Tech. 45 (1973), 1368; Rehm. Chem.Ing.Tech. 42 (1970), 583). Die Wirkung des Vibromixers beruht auf dem Bernoulli-Effekt. Anstelle eines Rührers befindet sich im Inneren des Gefässes eine waagerecht liegende, an einem senkrechten Schaft befindliche Platte, die sich nach unten verengende Bohrungen enthält. Sie wird mit Hilfe des Schafts auf und nieder bewegt. Dabei entstehen Druckdifferenzen in den Bohrungen, weil die Flüssigkeit in den Öffnungen vom großen zum kleinen Durchmesser zurückfließt. Die Zellen werden auf diese Weise zwar gut bewegt, doch bleiben Schädigungen weitgehend aus. Ein derartiger Vibromixer hat jedoch den Nachteil, daß er für die empfindlichen Pellets von Phanerochaete chrysosporium immer noch nicht schonend genug arbeitet. Außerdem können derartige Vibromixer bis heute noch nicht im großtechnischen Maßstab eingesetzt werden.

Für die Bildung von Pellets von Mikroorganismen sind schließlich auch Glasgefäße geeignet, welche von Schüttelmaschinen bewegt werden (H.J. Rehm: Einführung in die industrielle Mikrobiologie, Berlin-Heidelberg-New York 1971). Zu diesem Zweck sind die Behältnisse (zumeist Flaschen oder Erlenmeyerkolben) in mehreren Etagen übereinander in die Maschinen eingespannt. Durch die Schüttelbewegung der Gefäße wird Luft von der Oberfläche her in die Lösung eingeschlagen. Zum Schütteln verwendet man Amplituden-, Rotations- und Vibrationsmaschinen, die normalerweise variierbare Bewegungsgeschwindigkeiten haben. Die Gefäße können mit flächigen Ausbuchtungen, den sogenannten Schikanen, versehen sein. Nachteilig bei diesen Systemen ist, daß sie sich nur für den Labormaßstab eignen. Für die großindustrielle Anwendung wären tausende von Flaschen bzw. Erlenmeyerkolben notwendig, so daß die Anwendung derartiger Geräte wirtschaftlich nicht sinnvoll ist.

Um die obengenannten Probleme des Einsatzes von Rührreaktoren zu lösen hat man verstärkt versucht, mit immobilisierten Zellen zu arbeiten (S. Linko, Journal of Biotechnology, 8 (1988), 163-170; H. Willershausen, A. Jäger, H. Graf, Journal of Biotechnology, 6 (1987), 239-243; Y. Linko, M. Leisola, N. Lindholm, J. Troller, P. Linko, A. Fiechter, Journal of Biotechnology 4 (1986), 283-291).

Aber auch diese Versuchen scheiterten bei größeren Volumina. In der Regel waren diese Verfahren bei mehr als 40 1 Fermentervolumen nicht mehr durchführbar. Der Grund ist im wesentlichen darin zu sehen, daß Phanerochate chrysosporium in Pelletform eine optimale Oberfläche aufweist und daher gerade in diesem Zustand am besten das Enzym produziert.

Die vorliegende Erfindung hat sich daher die Aufgabe gestellt, ein Verfahren zur Herstellung lignolytischer Enzyme mittels Phanerochaete chrysosporium zur Verfügung zu stellen, das auch in Fermentationsgefäßen von mehr als 40 1 durchführbar ist.

Diese Aufgabe wird dadurch gelöst, daß Phanerochaete chrysosporium in ein rührerloses, geschlossenes Gefäß gegeben wird, dort unter Drehen und Schwenken des Gefässes Pellets von Phanerochaete chrysosporium erzeugt werden, diese dann bis zur Enzymproduktion weitergeschüttelt werden oder unmittelbar nach Bildung der Pellets in einen herkömmlichen Reaktor überführt wird und erst dort die Enzymproduktion stattfindet.

Die Bildung der Pellets erfolgt in einem neuartigen, für das erfindungsgemäße Verfahren speziell entwickelten Reaktor. Hierbei handelt es sich um ein rührerloses, geschlossenes Fermentationsgefäß, welches in einer kardanischen Aufhängevorrichtung frei dreh- und schwenkbar aufgehängt ist. Unter dem Gefäß ist ein Antriebsmotor angeordnet, dessen Antriebswelle mit dem Boden des Gefässes mit einem Exzenter verbunden ist.

Durch den Exzenter lassen sich Neigungswinkel und Schwingamplitude des Gefässes einstellen. Mittels des stufenlos regelbaren Motors können somit Dreh- und Schwenkbewegungen des Gefässes erreicht werden.

In dem auf diese Art bewegten Fermenter bildet Phanerochaete chrysosporium Pellets, ohne daß diese durch mechanische Einflüsse fortwährend wieder zerstört werden. Dies hat zur Folge, daß die Pellets eine für die Enzymproduktion optimale Form erreichen. Da andererseits die sehr schwingungsarme kardanische Aufhängung des Reaktors auch den Bau von Reaktorvolumina zwischen 1 und 3 m³ gestattet, läßt sich mit der beschriebenen Vorrichtung eine Produktion lignolytischer Enzyme im industriellen Maßstab mit bisher nicht für möglich gehaltenen Ausbeuten erreichen.

Als vorteilhaft ist weiterhin anzusehen, daß nach Abschluß der Enzymproduktion im Schüttelfermenter oder im Rührfermenter sich die abgetrennten Pellets von Phanerochaete chrysosporium unmittelbar wieder für die Enzymproduktion einsetzen lassen. Zweckmäßigerweise trennt man hierfür die Pellets von der restlichen Suspension ab und führt sie nach Abtrennung des Enzyms in den Rührreaktor zurück oder man gibt neues stickstoffarmes oder stickstofffreies Substrat ohne C-Quelle hinzu. Die Abtrennung kann erfindungsgemäß mittels Ultrafiltration durchgeführt werden.

Vor der Durchführung des erfindungsgemäßen Verfahrens kann Phanerochaete chrysosporium in bekannter Weise z.B. in Petrischalen oder in Standkulturen angezüchtet werden. Die gebildeten Mycele werden im Anschluß hieran zerschlagen und in dem erfindungsgemäßen Verfahren eingesetzt.

Die Anzucht von Phanerochaete chrysosporium erfolgt unter bekannten Bedingungen, d.h. es wird überlicherweise bei Temperaturen von 37 bis 40 °C und einem pH-Wert von 4,5 bis 5 gearbeitet. Der bevorzugte pH-Wert ist 5. Während der Anzucht von Phanerochaete chrysosporium kann kontinuierlich oder diskontinuierlich belüftet werden. Hierbei können entweder reiner Sauerstoff oder Pressluft verwendet werden.

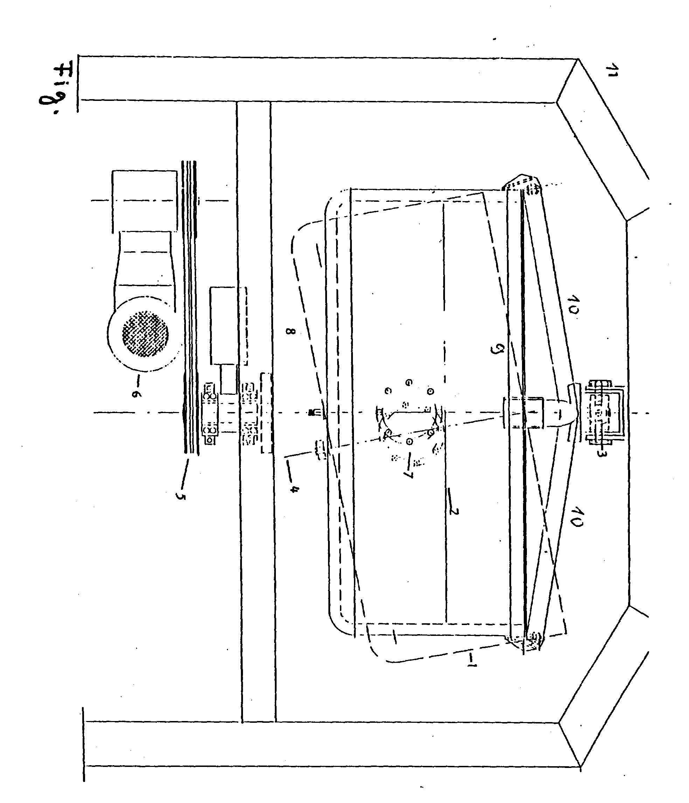

Im folgenden wird die Erfindung unter Bezugnahme auf die Figur näher beschrieben:

In der Figur ist der Reaktor dargestellt, welcher der Herstellung der Pellets von Phanerochaete chrysosporium dient. Hierbei erfolgt die Bildung der Pellets in dem Gefäß 1. Das Gefäß 1 ist ein geschlossener Behälter mit festem Boden 8, Seitenwänden, Deckel 9 und einer Sichtscheibe 7. Seitlich oder am Boden sind Öffnungen für diverse Meßfühler (O₂, PH, Antischaum) sowie für Zu- und Ablauf von Substrat und Impfkultur vorgesehen. Es ist in einem Gestell 11 aufgehängt. Die Befestigung erfolgt hierbei mittels der Greifarme 10. Über diese Greifarme 10 ist das Gefäß 1 an der kardanischen Aufhängvorrichtung frei dreh- und schwenkbar aufgehängt. Die Dreh- und Schwenkbewegungen werden durch den Motor 6 bewirkt. Dieser ist über die Antriebswelle S und den Exzenter 4 mit dem Boden 8 des Gefässes 1 verbunden. Mittels des Exzenters 4 werden Neigung und Schwingamplitude des Gefässes 1 eingestellt. Die Geschwindigkeit der Dreh- und Schwenkbewegungen des Gefässes 1 läßt sich über die Motordrehzahl regeln.

Die Erfindung wird weiterhin anhand des folgenden Beispiels erläutert:

### Beispiel

Als Stamm wird Phanerochaete chrysosporium ATCC 32629 genommen. Zunächst werden Malzagarplatten beimpft und ca. 10-12 Tage bei 27 °C bebrütet. Von diesen Platten wird die Hälfte des Rasens abgenommen und eine 50 ml Standkultur in 500 ml Erlenmeyerkolben beimpft (Bebrütungszeit ca. S Tage bei 37 °C). Die so erhaltene Vorkultur wird abdekantiert, wieder auf das ursprüngliche Volumen mit a.dest. aufgefüllt und daraufhin in einem Braun Starmix für 2 x 30 Sec. auf Stufe 3 zerkleinert. Pro Liter Medium im Schüttelreaktor werden 15 ml Vorkultur zugegeben, d.h. auf einen 10 l Schüttelreaktor mit 5 l Befüllung 75 ml. Die Anzuchttemperatur beträgt 38 °C, die Schüttelfrequenz 90 rpm und die Auslenkung ca. 5 cm. Es wird jeden Tag für 30 Sec. mit O₂ (100 l pro Std.) begast. Die Anzuchtdauer beträgt 4-5 Tage. Die Enzymausbeuten betragen ca. 50 - 100 U/l (1 U = 1µ Mol Umsatz von Veratrylalkohol in Veratryldehyd/ Min.). Bei einem konventionellen Rührreaktor sind unter gleichen Bedingungen höchstens 10 U/l zu erreichen.

Die Medien sind wie folgt zusammengesetzt:

### Vorkulturmedium:

### 1) Mineralsalzlösung (ME): (pro Liter)

10,5 g Nitrilotriacetat; 21 g MgSO₄ , 3,5 g MnSO₄ ; 7 g NaCl, 0,7 g FeSO₄ ; 0,7 g CoCl₂ ; 0,7 g CaCl₂ ; 0,7 g ZnSO₄ , 0,07 g CuSO₄ ; 0,07 g CuSO₄ ; 0,07 g AlK₂SO₄ ; 0,07 g H₃BO_{S} ; 0,07 g NaMoO₄.

### 2) Lösung 1 (Salzlösung) (pro Liter)

20 g KHyPO₄ ; 5 g MgSO₄ ; 1 g CaCl₂.

### 3) Puffer (pH 4,5-S,S)

1 m NaH₂PO₄ /Na₂HPO₄, - Puffer.

Zusammensetzung pro 1 l Medium:

| | |
|---|---|
| 0,2 g | Ammoniumtartrat |
| 100 ml | Lösung 1 |
| 1,43 ml | ME-Lösung |
| 10 g | Glukose |
| 10 ml | Puffer |
| + 0,9 ml | Thiamin 100 mg/l (wird nach dem Autroklavieren sterifiltriert zugesetzt) |

### Hauptkulturmedium:

Zusammensetzung pro 1 Medium :

| | |
|---|---|
| 0,2 g | Ammoniumtartrat |
| 100 ml | Lösung 1 |
| 10 ml | ME-Lösung |
| 10 g | Glukose |
| 10 ml | Puffer |
| 67,3 mg | Veratrylalkohol |
| 2 ml | Tween 80 |
| + 0,9 ml | Thiamin 100 mg/l |

## Patentansprüche

1. Verfahren zur Herstellung lignolytischer Enzyme mittels Phanerochaete chrysosporium,
**dadurch gekennzeichnet**, daß
a) eine Kultur des Weißfäulepilzes Phanerochaete chrysosporium in ein rührerloses, geschlossenes Gefäß (1) gegeben wird,
b) dort unter Drehen und Schwenken des Gefässes (1) Pellets von Phanerochaete chrysosporium erzeugt werden und
c) danach Enzym geerntet wird.

2. Verfahren nach Anspruch 1,
**dadurch gekennzeichnet,** daß die die Pellets enthaltende Suspension vor der Enzymernte in einen herkömmlichen Rührreaktor überführt wird und dort die Enzyme mittels der Pellets erzeugt werden.

3. Verfahren nach Anspruch 1,
**dadurch gekennzeichnet**, daß nach Abschluß der Enzymproduktion die Pellets vom Medium abgetrennt und erneut zur Enzymproduktion in dem Rührreaktor eingesetzt werden.

4. Verfahren nach Anspruch 1 oder 2,
**dadurch gekennzeichnet**, daß Phanerochaete chrysosporium nicht in Reinkultur vorliegt.

5. Verfahren nach einem der Ansprüche 1 bis 4,
**dadurch gekennzeichnet,** daß zunächst Phanerochaete chrysosporium in an sich bekannter Weise in Standkulturen oder auch auf Platten angezüchtet und anschließend das Verfahren mit zerschlagenem Mycel von Phanerochaete chrysosporium durchgeführt wird.

6. Verfahren nach einem der Ansprüche 1 bis 5,
**dadurch gekennzeichnet**, daß die Kultur mit Sauerstoff oder Pressluft belüftet wird.

7. Vorrichtung zur Erzeugung von Pellets des Pilzes Phanerochaete chrysosporium für die Verwendung in dem Verfahren gemäß einem der Ansprüche 1 bis 6,
**dadurch gekennzeichnet**, daß sie aus einem geschlossenen, rührerlosen Fermentationsgefäß (2) besteht, welches in der kardanischen Aufhängvorrichtung (3) frei dreh- und schwenkbar aufgehängt ist.

8. Vorrichtung nach Anspruch 7,
**dadurch gekennzeichnet,** daß zwecks Bewirkung von Dreh- und Schwenkbewegungen des Gefässes (1) unter dessen Boden (8) ein Motor (6) angeordnet ist, dessen Antriebswelle (5) mit dem Boden (8) des Gefässes (1) über einen Exzenter (4) verbunden ist.

9. Vorrichtung nach Anspruch 8,
**dadurch gekennzeichnet,** daß die Motordrehgeschwindigkeit stufenlos einstellbar ist.

10. Vorrichtung nach Anspruch 8,
**dadurch gekennzeichnet,** daß der Neigungswinkel und die Schwingungsamplitude des Gefässes (1) mittels des Exzenters (4) verstellbar sind.

## Claims

1. A process for the production of lignolytic enzymes by means of phanerochaete chrysosporium,
characterised in that
a) a culture of the white putrefactive fungus phanerochaete chrysosporium is provided in a closed container (1) with no agitator,
b) pellets of phanerochaete chrysosporium are produced there during rotation and oscillation of the container (1) and
c) thereafter enzyme is collected.

2. A process according to Claim 1, characterised in that the suspension containing the pellets is transferred before the collection of enzyme into a conventional agitation reactor and there the enzymes are produced by means of the pellets.

3. A process according to Claim 1, characterised in that after termination of the enzyme production the pellets are separated from the medium and are once again used for the enzyme production in the agitation reactor.

4. A process according to Claim 1 or 2, characterised in that phanerochaete chrysosporium is not present in a pure culture.

5. A process according to one of claims 1 to 4, characterised in that firstly phanerochaete chrysosporium is cultivated in a manner known per se in habitat cultures or even on dishes and subsequently the process is carried out with a broken mycelium of phanerochaete chrysosporium.

6. A method according to one of claims 1 to 5, characterised in that the culture is aerated with oxygen or compressed air.

7. A device for the production of pellets of the fungus phanerochaete chrysosporium for use in the method according to one of claims 1 to 6, characterised in that it consists of a closed fermentation container (2) with no agitator, which is suspended in the cardanic suspension device (3) so as to be capable of free rotation and oscillation.

8. A device according to claim 7, characterised in that with the aim of effecting rotational and oscillatory movements of the container (1), a motor (6) is arranged below the base thereof, the drive shaft (5) of which motor is connected to the base of the container via an eccentric (4).

9. A device according to claim 8, characterised in that the rotational speed of the motor is steplessly adjustable.

10. A device according to claim 8, characterised in that the angle of inclination and the amplitude of the oscillation of the container (1) are adjustable by means of the eccentric (4).

## Revendications

1. Procédé de préparation d'enzymes lignolytiques à l'aide de Phanerochaete chrysosporium, caractérisé en ce que
(a) on place dans une cuve fermée (1), sans agitateur une culture du champignon de la pourriture blanche 5 Phanerochaete chrysosporium,
(b) on y produit, par rotation et pivotement de la cuve (1) des pastilles de Phanerochaete chrysosporium, et
(c) puis on cultive l'enzyme.

2. Procédé selon la revendication 1, caractérisé en ce 10 que la suspension contenant les pastilles est, avant la récolte des enzymes, transvasée dans un réacteur classique muni d'un agitateur, les enzymes y étant produites à l'aide des pastilles.

3. Procédé selon la revendication 1, caractérisé en ce 15 que, après la fin de la production des enzymes, on sépare les pastilles du milieu et on les utilise de nouveau pour la production d'enzymes dans le réacteur muni d'un agitateur.

4. Procédé selon la revendication 1 ou 2, caractérisé en ce que Phanerochaete chrysosporium ne se présente pas sous 20 forme de culture pure.

5. Procédé selon l'une des revendications 1 à 4, caractérisé en ce qu'on cultive d'abord Phanerochaete chrysosporium d'une manière connue en soi, en culture verticale ou sur plaques, puis on met en oeuvre le procédé, 25 avec un mycellium brisé de Phanerochaete chrysosporium.

6. Procédé selon l'une des revendications 1 à 5, caractérisé en ce que la culture est aérée par de l'oxygène ou de l'air comprimé.

7. Dispositif pour fabriquer des pastilles du champignon 30 Phanerochaete chrysosporium pour utilisation dans le procédé selon l'une des revendications 1 à 6, caractérisé en ce qu'il est constitué d'une cuve de fermentation (2) fermée, sans agitateur, qui est suspendue dans le dispositif de suspension à cardan (3) en pouvant subir librement un 35 mouvement de rotation et de pivotement.

8. Dispositif selon la revendication 7, caractérisé en ce que, pour permettre des mouvements de rotation et de pivotement de la cuve (1), un moteur (6), dont l'arbre d'entraînement est relié au fond (8) de la cuve (1) par l'intermédiaire d'un excentrique (4), est disposé en dessous 5 du fond (8).

9. Dispositif selon la revendication 8, caractérisé en ce que la vitesse de rotation du moteur peut être ajustée d'une manière continue.

10. Dispositif selon la revendication 8, caractérisé en 10 ce que l'angle d'inclinaison et l'amplitude d'oscillation de la cuve (1) sont réglables à l'aide de l'excentrique (4).
